# EUROPEAN PATENT APPLICATION

(11) **EP 4 353 825 A1**
(43) Date of publication of application: **17.04.2024**
(21) Application number: 22200620.7
(22) Date of filing: 10.10.2022
(51) Int. Cl.: C12N 15/85

(54) **MODIFIED PROMOTER SEQUENCES**

(71) Applicant: bisy GmbH, 8200 Hofstätten an der Raab (AT)
(72) Inventor: Glieder, Anton, 8010 Graz (AT); Hussein, Mohamed, 8010 Graz (AT); Hatzl, Anna-Maria, 8010 Graz (AT); Ebner, Katharina, 8010 Graz (AT)
(74) Representative: Schwarz & Partner Patentanwälte GmbH

(57) **Abstract**

The present invention relates to a method for modifying a promoter to obtain a promoter variant comprising the steps of:
a. providing a promoter,
b. modifying the promoter
- by inserting at least one nucleic acid sequence selected from the group consisting of GATAX₁X₂X₃X₄X₅X₆(X₇)ₘ, ATCCTTTTAG (SEQ ID No. 1) and AAA into the promoter and/or
- by substituting nucleotides of the promoter sequence to introduce at least one nucleic acid sequence selected from the group consisting of GATAX₁X₂X₃X₄X₅X₆(X₇)ₘ, ATCCTTTTAG (SEQ ID No. 1) and AAA into the promoter,

wherein X₁, X₂, X₃, X₄, X₅, X₆, and X₇ are nucleotides independently selected from the group consisting of A, C, G and T, and wherein m is an integer between 0 and 10.

## Description

### TECHNICAL FIELD

The present invention relates to the technical field of promoters, in particular promoter variants showing an enhanced gene expression level.

### BACKGROUND OF THE INVENTION

At the most fundamental level protein expression is restricted by the amount of transcript available in the cells for translation. The overall transcript levels depend on the gene copy number and the number of transcripts produced, which in turn are strongly influenced by the promoter. Due to this direct impact on expression promoter sequences are among best studied regulatory elements of eukaryotes and promoter engineering is used as a way to fine-tuning gene expression.

The engineering of promoter sequences is a broad field and especially for eukaryotic promoters mostly focused on modification of already existing sequences. Deletions and base exchanges of short DNA stretches, as well as truncations provide opportunities to improve promoter performance by genetic engineering. The modification of promoter most of the time aims the creation of especially strong promoter sequences to push transcription to its maximum.

Engineering approaches can be random, rational or a combination of both. Although random methods are more likely to result in a large number of variants with decreased strength, they require far less knowledge and understanding about/of the modified sequence. So far, saturation mutagenesis of spacer regions, random mutagenesis by error prone PCR, hybrid promoter engineering and direct modification of transcription factor binding sites (TFBS) are used in promoter engineering. The fundamental aim of all these strategies is to influence transcription by changing the TFBS of a promoter sequence, either single ones specifically, the combination of them in the sequence or the context of their occurrence. However, based on analysis of expression also alternative effects have to be kept in mind as potential reasons for changes in target-protein yield, which are differences in mRNA stability or translational initiation.

Hence, it is an object of the present invention to provide methods for obtaining promoters exhibiting superior properties compared to the respective wild-type promoter from which they are derived from. Another object of the present invention is the provision of promoters having superior properties compared to the respective wild-type promoter from which they are derived from.

### SUMMARY OF THE INVENTION

The present invention relates to a method for modifying a promoter to obtain a promoter variant comprising the steps of:
a. providing a promoter,
b. modifying the promoter
   - by inserting at least one nucleic acid sequence selected from the group consisting of GATAX₁X₂X₃X₄X₅X₆(X₇)ₘ, ATCCTTTTAG (SEQ ID No. 1) and AAA into the promoter and/or
   - by substituting nucleotides of the promoter sequence to introduce at least one nucleic acid sequence selected from the group consisting of GATAX₁X₂X₃X₄X₅X₆(X₇)ₘ, ATCCTTTTAG (SEQ ID No. 1) and AAA into the promoter,
wherein X₁, X₂, X₃, X₄, X₅, X₆, and X₇ are nucleotides independently selected from the group consisting of A, C, G and T, and wherein m is an integer between 0 and 10.

Surprisingly, it has been found that the insertion of "neutral" nucleic acid sequences such as GATAX₁X₂X₃X₄X₅X₆(X₇)ₘ, ATCCTTTTAG (SEQ ID No. 1) and/or AAA into a promoter is useful to obtain promoter variants showing an increased transcription rate compared to the respective wild-type promoter. These neutral nucleic acid sequences might have the advantage to not influence the binding of transcription factors to the promoter in a cell. Furthermore, the use of neutral nucleic acid sequences might prevent the formation of new transcription factor binding sites having unpredictable effects on the transcription in a cell. It was found that nucleic acid sequences GATAX₁X₂X₃X₄X₅X₆ (X₇) ₘ, ATCCTTTTAG (SEQ ID No. 1) and AAA are to be considered as neutral nucleic acid sequences. These sequences were identified to not comprise possible binding sites for eukaryotic transcription factors. These nucleic acid sequences turned out to be very useful in the identification and production of promoter variants having superior effects compared to the unmodified promoter. These nucleic acid sequences can be longer or shorter but preferably contain 10 bases, which might support a neutral effect on the 3-dimensional structure of the promoter DNA including bound transcription factors, in case the insertion does not replace a sequence of identical length. These nucleic acid sequences can be used to obtain promoters with a higher promoter activity compared to the unmodified promoter. Hence, the method of the present invention is suited for identifying and/or obtaining promoters/promoter variants showing an enhanced promoter activity, in regard to protein expression, for instance, compared to an unmodified promoter. The present invention relates therefore also to a method for identifying promoter variants showing an enhanced promoter activity compared to an unmodified promoter and/or for screening a library of promoter variants modified by inserting at least one nucleic acid sequence selected from the group consisting of GATAX₁X₂X₃X₄X₅X₆ (X₇) ₘ, ATCCTTTTAG (SEQ ID No. 1) and AAA into a promoter and/or by substituting nucleotides of the promoter sequence to introduce at least one nucleic acid sequence selected from the group consisting of GATAX₁X₂X₃X₄X₅X₆(X₇)ₘ, ATCCTTTTAG (SEQ ID No. 1) and AAA into a promoter. In both cases the promoter variants can operably linked to a target sequence to be transcribed and the amount of transcription product (i.e. RNA or protein/polypeptide) compared to a unmodified promoter shows whether the promoter activity is increased or decreased compared to the said unmodified promoter.

The promoter to be modified by the method of the present invention may be of any origin. Thus, the promoter may be a human or animal, a bacterial, a fungal, a viral or an artificial promoter. The method of the present invention can also be used to introduce modifications into promoters which have already been modified or optimized, respectively. The claimed method is a further tool to improve such promoters in regard to transcription rate or other properties.

Another aspect of the present invention relates to a promoter variant obtainable by a method according to the present invention.

Promoters obtainable with the method of the present invention may show a much higher transcription rate compared to the respective unmodified promoter from which they are derived from. Promoters obtainable with the method of the present invention may cause a much higher protein yield or specific productivity compared to the respective unmodified promoter from which they are derived from. The promoters obtained by the method of the present invention can be considered as promoter variants.

A further aspect of the present invention relates to a nucleic acid molecule or a vector comprising a promoter variant of the present invention or a promoter variant obtainable by the method of the present invention.

Nucleic acid molecules according to the present invention may comprise a promoter according to the present invention and a nucleic acid sequence encoding for a protein or polypeptide operably linked thereto. Thus, the transcription of the encoding region of such a nucleic acid molecule is controlled by the promoter. Such nucleic acid molecules or a promoter variant of the present invention may be comprised in a vector. The vector containing the nucleic acid molecule of the present invention or the promoter of the present invention may be a linear expression construct for genome integration, a plasmid, a cosmid, or a viral vector.

Another aspect of the present invention relates to a host cell comprising a promoter variant, nucleic acid molecule or vector according to the present invention. The host cell containing the promoter variant according to the invention may be a human, an animal, a plant or a fungal cell. The promoter introduced into these cells directly or via a nucleic acid molecule and/or vector according to the present invention shall be functional active in these cells and, hence, comprising respective functional elements.

A further aspect of the present invention relates to a method for producing a polypeptide or protein comprising the step of cultivating a host cell comprising a promoter variant obtainable by the method of the present invention, a nucleic acid molecule or a vector according to the present invention. The nucleic acid sequence encoding said polypeptide or protein is operably linked to the promoter of the present invention allowing its expression in the host cell and optionally his secretion from said host cell.

### SHORT DESCRIPTION OF THE FIGURES

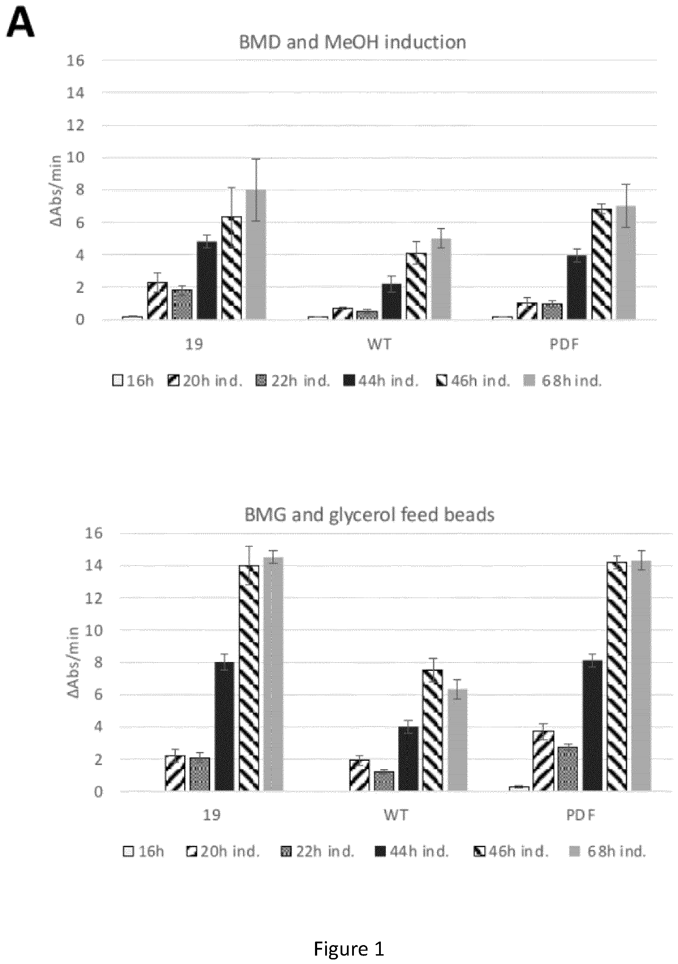

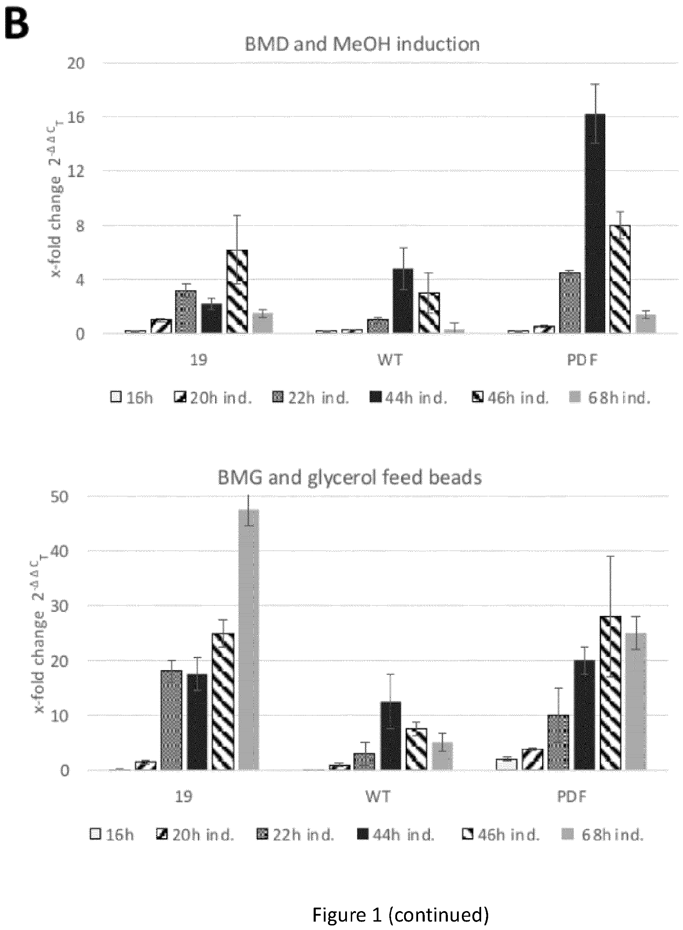

Fig. 1 shows lipase activity (A) and fold changes in the target gene (CalB) (B) for PCAT variant #19, the original PCAT promoter and reference promoter PDF.

### DETAILED DESCRIPTION

The method according to the present invention is designed to modify a promoter using "neutral" nucleic acid sequences. "Neutral" nucleic acid sequences refer to nucleic acid molecules which themselves do not comprise any properties which may influence the activity of a promoter. This means that these neutral nucleic acid sequences do neither comprise a transcription factor binding site (TFBS) and/or repressor or activator protein binding sites nor are they able to form a TFBS and repressor and activator protein binding sites together with any other nucleic acid sequence present in the promoter to be modified. Thus, neutral nucleic acid sequences are advantageous since they are not able to form a new TFBS into a promoter sequence which may have undesired effects on the promoter activity.

The neutral nucleic acid sequences can be introduced into the promoter by insertion or substitution. These nucleic acid sequences can be introduced into the promoter at one or more sites of the promoter, whereby it is preferred to introduce them into 1 to 10 sites, preferably 1 to 8 sites, more preferably 1 to 6 sites, more preferably 1 to 4 sites, more preferably 1 to 3 sites, more preferably 1 or 2 sites, of the promoter. In order to identify modified promoters exhibiting altered properties compared to the unmodified promoter, the one or more neutral nucleic acid sequences may be introduced randomly into the promoter sequence. Alternatively, it is also possible to introduce them systematically step by step from the 5' end to the 3' end of the promoter. The introduction can be an additional insertion into the promoter sequence or replace a part of the original promoter sequence by substitution. The modified promoters obtained by both approaches can be operably linked to a nucleic acid molecule encoding for a polypeptide or protein and introduced in a host cell to measure the polypeptide or protein expression rate. Alternatively, it is also possible to determine the amount of RNA, in particular mRNA, transcribed under the control of the promoter variant obtainable by the inventive method and the unmodified promoter. An increase or decrease of the protein and/or polypeptide expression rate and/or the transcription rate indicates whether a specific modification results in a modified promoter showing an increased or decreased expression/transcription rate and, thus, promoter activity. Hence, the method of the present invention can also be defined to be a method for identifying promoter variants showing an increased or decreased expression rate when operably linked to a nucleic acid sequence encoding a polypeptide or protein, for instance.

The term "operably linked", as used herein, refers to a linkage of polynucleotide elements in a functional relationship. A nucleic acid is "operably linked" when it is placed into a functional relationship with another nucleic acid sequence. A promoter is operably linked to a coding sequence if it affects the transcription of the coding sequence. Operably linked may mean that the nucleic acid sequences being linked are contiguous.

"Promoter", as used herein, refers to a nucleic acid fragment that functions to control the transcription of one or more nucleic acid molecules. A promoter is typically located upstream (5') with respect to the direction of transcription of the transcription initiation site of the gene and is structurally identified by the presence of a binding site for DNA-dependent RNA polymerase, transcription initiation site(s) and can further comprise any other nucleic acid sequences, including, but not limited to transcription factor binding sites, repressor and activator protein binding sites and any other sequences of nucleotides known to one of skill in the art to act directly or indirectly to regulate the amount of transcription from the promoter. A "promoter variant" is a promoter being modified by the method of the present invention.

Insertion of at least one nucleic acid sequence is understood to mean the insertion of a nucleic acid sequence into an existing promoter sequence. The sequence can be inserted at any position in the promoter sequence. The insertion of the nucleic acid sequence merely adds nucleotides to the original promoter sequence; no nucleotides of the original promoter sequence are removed. By this modification, the length of the promoter sequence changes by the number of nucleotides inserted by insertion. The positions of the nucleotides located before the insertion site remain unchanged. The positions of the nucleotides located after the insertion site change by the number of nucleotides inserted. If a multiple insertion takes place, the positions of the individual nucleotides are determined by adding the number of nucleotides inserted before them to the original position.

Substitution of nucleotides of a promoter sequence in order to insert at least one nucleic acid sequence is the exchange of a part of the existing promoter sequence with a nucleic acid sequence. During substitution, the same number of nucleotides of the original promoter sequence is removed as new ones are added. This does not change the position of the individual nucleotides both upstream and downstream. Since the promoter length may have an influence on its activity and overall behaviour it is preferred that the modification of the promoter occurs via substitution so that the promoter is modified by substituting nucleotides of the promoter sequence to introduce at least one nucleic acid sequence selected from the group consisting of GATAX₁X₂X₃X₄X₅X₆(X₇)ₘ, ATCCTTTTAG (SEQ ID No. 1) and AAA into the promoter.

In the first step of this method for modifying a promoter, a promoter must be provided. This promoter can be of any origin. The promoter can be a naturally occurring promoter, but it can also be a variant of such a naturally occurring sequence. Hybrid versions of several naturally occurring promoters are also suitable. A synthetically produced promoter sequence can also be used. Persons skilled in the art know how to produce such sequences.

One of the neutral nucleic acid sequences that can be used in the method of the present invention consists of the nucleic acid sequence GATAX₁X₂X₃X₄X₅X₆(X₇)ₘ. This nucleic acid sequence can be used to modify the original promoter by insertion or substitution. X₁ to X₇ stand for nucleotides. The nucleotides are preferably selected from the group consisting of A, C, G, and T.

According to a particularly preferred embodiment of the present invention X₁ is A or T, X₂ by C or G, X₃ by C or G, X₄ by C or G, X₅ by A or T and X₆ by C or G, whereby X₁ is even more preferred A, X₂ is even more preferred C, X₃ is even more preferred C, X₄ is even more preferred G, X₅ is even more preferred T and X₆ is even more preferred G.

Nucleic acid sequence GATAX₁X₂X₃X₄X₅X₆(X₇)ₘ may consist of 10 to 20 nucleotides, whereby variable m defines the length of said nucleic acid sequence. m is defined to be an integer between 0 and 10, wherein in a preferred embodiment of the present invention m is an integer between 0 and 8, more preferably 0 and 6, more preferably between 0 and 4, more preferably between 0 and 2. In a particular preferred embodiment m is 0, so that the nucleic acid sequence to be incorporated into a promoter has a length of 10 nucleotides. The variation in the length of the nucleic acid sequence also varies the number of substituted nucleotides in the promoter.

In a particularly preferred embodiment of the present invention the nucleic acid sequence to be incorporated into a promoter consists of GATAACCGTG (SEQ ID No. 2). This nucleic acid sequence can be either inserted into the promoter or substituted by nucleotides of the promoter.

The nucleic acid sequence to be incorporated into a promoter by insertion and/or substitution may alternatively consist of ATCCTTTTAG (SEQ ID No. 1) and/or AAA. Also these nucleic acid sequences are to be considered as neutral nucleic acid sequences and may lead to modified promoters showing an altered, preferably an increased, transcription rate of a nucleic acid molecules encoding a polypeptide or protein.

In a particularly preferred embodiment of the present invention the promoter may be modified by incorporating (by substation or insertion) various combinations of nucleic acid sequences GATAX₁X₂X₃X₄X₅X₆ (X₇) ₘ, ATCCTTTTAG (SEQ ID No. 1) and AAA. It is particularly preferred to incorporate GATAX₁X₂X₃X₄X₅X₆(X₇)ₘ and ATCCTTTTAG (SEQ ID No. 1), GATAX₁X₂X₃X₄X₅X₆ (X₇) ₘ and AAA, ATCCTTTTAG (SEQ ID No. 1) and AAA or even all three of these nucleic acid sequences (i.e. GATAX₁X₂X₃X₄X₅X₆(X₇)ₘ, ATCCTTTTAG (SEQ ID No. 1) and AAA).

According to an especially preferred embodiment of the present invention the promoter is modified in the regulatory region of the promoter. The modification in the regulatory region of the promoter makes it possible to identify potential regulatory hotspots and to manufacture modified promoters with altered regulation profiles and increased promoter strength. A "regulatory region" of a promoter comprises or consists of a TFBS and/or suppressor binding site. Transcription factor binding sites are regions within the promoter that influence transcription. Regulatory regions can be identified using a semi-rational approach. Suppressor binding sites bind polypeptides or proteins which suppress and thus reduce the transcription rate of the promoter. Modifications of the promoter sequence either upstream or downstream of the TFBS or suppressor binding site is particularly preferred.

According to another preferred embodiment of the present invention the promoter is a eukaryotic promoter, i.e. a promoter which is directly derived from an eukaryotic cell or functionally active in a eukaryotic cell. In an even more preferred embodiment of the present invention the promoter to be modified is a fungal promoter. A fungal promoter is a promoter naturally present in cells of the fungi kingdom or functionally active in such cells. Particularly preferred fungal promoters are yeast promoters, more preferably promoters of methylotrophic yeast cells.

According to an even more preferred embodiment of the present invention the fungal promoter is a promoter of a fungal cell from the phylum Ascomycota. Ascomycota are of great importance to humans, as they are responsible for many diseases and are also relevant in medicine, food industry and ecosystems. Some representatives of Ascomycota are used for manufacturing recombinant proteins and polypeptides.

The promoter of a fungal cell from the phylum Ascomycota is preferably from the genus Komagataella, more preferably from *Komagataella phaffii.* Other preferable promoters are DNA sequences which are active as promoters in *Trichoderma* sp, *Myceliophthora* sp. and *Aspergillus* sp.

Due to its large popularity, strength and tight regulation, the alcohol oxidase 1 promoter of *Komagataella phaffii* (*Pichia pastoris*) is a prime example for promoter engineering. Using a random engineering approach variants with increased depressed expression and higher expression could be produced.

*Komagataella phaffi* is one of the most important eukaryotic hosts for recombinant protein production with its capacity to produce and secrete proteins to high levels, glycosylation pattern and low amount of endogenously secreted proteins. The classic promoter sequences for the *Komagataella phaffii* expression system are still used frequently. New alternatives include promoters of genes associated with the methanol utilization pathway. As such the catalase promoter of *Komagataella phaffii* is described, which is not only inducible by methanol, but also got activated upon carbon source depletion called derepression increasing the feasibility of methanol-free protein production.

The modified promoter according to the present invention can be used to increase the expression of proteins or polypeptides in fungi, preferably *Komagataella phaffi.* In particular, the modified promoter of the present invention can increase the protein expression of antibodies and other binding proteins or fragments thereof, enzymes, food and feed proteins, biopharmaceutical proteins, structural proteins and proteins for materials and cosmetics

Still further preferred the promoter according to the present invention is a yeast promoter, preferably associated with a gene encoding for a catalase, preferably for a catalase from *Komagataella phaffii,* or a heat shock protein, preferably for heat shock protein 12, more preferably for heat shock protein 12 from *Komagataella phaffii.*

*K. phaffii* is one of the most important eukaryotic hosts for recombinant protein production with its capacity to produce and secrete proteins to high levels, favourable glycosylation pattern and low amount of endogenously secreted proteins, to name only a few advantages. Although it has been shown that fine-tuning gene expression by employing promoter sequences with suitable regulation elements and strengths is a feasible approach to increase the yield for your protein(s) of interest, the classic promoter sequences for the *K. phaffii* (PAOX1 and PGAP) expression system are still used frequently. Alternatives include promoters of genes associated with the MUT pathway. There, for the first time catalase promoters of *K phaffii,* e.g. PCTA1 (also known as PCAT), were described as a strong regulated promoter with intriguing response to various carbon sources. These promoters are not only inducible by methanol, but also can be activated upon carbon source depletion, a mechanisms known from regulatory sequences associated with the carbon metabolism and called derepression. Due to the favourable qualities of the PCTA1 it was chosen as a target for promoter engineering to identify potential regulatory regions and to create novel sequence-diversified promoter variants.Another very strong regulatory sequence is the promoter of heat shock protein (HSP12), named PDH, which may also serve a promoter to be modified with the method of the present invention.

Hence, the promoter used in the present invention preferably comprises or consists of a nucleic acid sequence having at least 80% identity, preferably at least 90% identity, more preferably at least 95% identity, to SEQ ID No. 3 or SEQ ID No. 4, wherein it is particularly preferred that the promoter used in the present invention comprises or consists of nucleic acid sequence SEQ ID No. 3 or SEQ ID No. 4.
PCTA1 (SEQ ID No. 3): 
TAATCGAACTCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGGTCGGCATTACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTCTCGCACGAGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGTT
TCATTATCCCAGCGGGGTGATAGCCTCTGTTGCTCATCAGGCCAAAATCATATATAAGCTGT
AGACCCAGCACTTCAATTACTTGAAATTCACCATAACACTTGCTCTAGTCAAGACTTACAAT
TAAA
PDH (SEQ ID No. 4):

CTTCAGTAAGAGAAGGAAGCTTGGTGACGATCACAGCTATGATGTAATAGAAATTGCTAAGC
AATTGTGAGGTGTGATGTATTTTGCAGAGCAATTGTGCGGTACAACGGGGTGTTATTGTCTT
CACAAGGCATTTATTGCGAATTTCGTAGTTGAAAGAATATTTTAGCACAGGGTGCTTGACCC
CTATTGTTGCTCGCTAAACCATGATTGCTAAATGATGACATAGCAATCACTTTACTAAGATT
GCTATAAGGACACCTTTCTTAGTATAAATGGACACTCTTTTCCCCTGCTAAACTTCTTTTAT
TTTTCACACTTAAACAGTTACAAAACACAAACACAACTAGAA

"Identity" of two or more nucleic acid sequences can be determined by aligning these nucleic acid sequences. An alignment can be performed using BLAST (National Center for Biological Information (NCBI) Basic Local Alignment Search Tool) version 2.13.0 software with default parameters. Nucleic acid % sequence identity between nucleic acid sequences can be determined using standard nucleotide BLAST with the following default parameters: Max target sequences: 100; Short queries: Automatically adjust parameters for short input sequences; Expect threshold: 10; Word size: 28; Max matches in a query range: 0; Match/Mismatch Scores: 1, -2; Gap costs: Linear; Filter: Low complexity regions; Mask: Mask for lookup table only. A sequence having an identity score of XX % (for example, 80%) with regard to a reference sequence using the NCBI BLAST version 2.13.0 algorithm with default parameters is considered to be at least XX % identical or, equivalently, have XX % sequence identity to the reference sequence.

The modifications described below for promoter comprising or consisting of a nucleic acid sequence having at least 80% identity to SEQ ID No. 3 or SEQ ID No. 4 are preferably substitutions of nucleotides of the promoter sequence to introduce at least one nucleic acid sequence selected from the group consisting of GATAX₁X₂X₃X₄X₅X₆(X₇)ₘ, ATCCTTTTAG (SEQ ID No. 1) and AAA into the promoter.

The promoter comprising or consisting of a nucleic acid sequence having at least 80% identity to SEQ ID No. 3 can be modified according to the present invention. The modification of the promoter according to the invention leads to a promoter exhibiting improved properties compared to the wild-type/unmodified promoter, in particular it leads to an increased transcription efficiency and, thus, an increased protein expression. The promoter can be improved even more by carrying out multiple modifications according to the present invention.

According to a particularly preferred embodiment of the present invention the promoter comprising or consisting of a nucleic acid sequence having at least 80% identity to SEQ ID No. 3 is modified within at least one promoter region selected from the group consisting of position 1 to 20, position 131 to 150, position 251 to 270, position 371 to 390, position 401 to 410 and position 461 to 480 of SEQ ID No. 3. Even more preferred the promoter comprising or consisting of a nucleic acid sequence having at least 80% identity to SEQ ID No. 3 is modified within at least one promoter region selected from the group consisting of position 1 to 10, position 131 to 140, position 251 to 260, position 371 to 380, position 401 to 410 and position 471 to 480 of SEQ ID No. 3. In an even more preferred embodiment the position 1 to 10 is thereby preferably modified with ATCCTTTTAG (SEQ ID No. 1) and positions 131 to 140, 251 to 260 371 to 380, position 401 to 410 and 471 to 480 are preferably modified with GATAX₁X₂X₃X₄X₅X₆(X₇)ₘ.

The modification of the promoter comprising or consisting of a nucleic acid sequence having at least 80% identity to SEQ ID No. 3 at one or more of the aforementioned sites by introducing at least one nucleic acid sequence selected from the group consisting of GATAX₁X₂X₃X₄X₅X₆(X₇)ₘ, ATCCTTTTAG (SEQ ID No. 1) and AAA or substituting nucleotides of the promoter to introduce at least one nucleic acid sequence selected from the group consisting of GATAX₁X₂X₃X₄X₅X₆(X₇)ₘ, ATCCTTTTAG (SEQ ID No. 1) and AAA results in a promoter variant showed a significantly increased transcription rate compared to the wild-type promoter, whereby nucleic acid sequence GATAX₁X₂X₃X₄X₅X₆(X₇)ₘ is preferably used for the promoter modifications.

According to another especially preferred embodiment of the present invention the promoter comprising or consisting of a nucleic acid sequence having at least 80% identity to SEQ ID No. 3 is modified simultaneously at positions 1 to 10, 131 to 140 and 251 to 260; or 131 to 140, 251 to 260 and 401 to 410; or 131 to 140, 251 to 260 and 471 to 480; or 1 to 10, 131 to 140, 251 to 260 and 371 to 380; or 1 to 10, 131 to 140, 251 to 260 and 471 to 480; or 1 to 10, 131 to 140, 251 to 260, 371 to 380, 401 to 410 and 471 to 480.

According to the present invention promoters can also be modified additionally by deletion. Modifying a promoter sequence by deletion means that certain nucleotides of the original promoter sequence are eliminated. No new nucleotides are added to the promoter sequence. The deletion of single nucleotides or nucleic acid stretches within the promoter to be modified, may also influence the transcription rate of nucleic acid sequences operably linked to such promoters. Hence, it is particularly preferred to delete one or more, preferably one, two, three, four, five, six or even all, nucleotides within positions 89 to 101, 137, 138, 162, 176 and/or 448 to 468 of the promoter comprising or consisting of a nucleic acid sequence having at least 80% identity to SEQ ID No. 3. Deletions on putative transcription factor binding sites are particularly preferred.

According to an especially preferred embodiment of the invention the promoter comprising or consisting of a nucleic acid sequence having at least 80% identity to SEQ ID No. 3 is modified at positions 371 to 380 by substitution and at positions 89 to 101, 137, 138, 162 and 176 by deletion, or at positions 251 to 260 by substitution and at positions 89 to 101, 137, 138, 162 and 176 by deletion, or at positions 131 to 140 and 251 to 260 by substitution and at positions 448 to 468 by deletion, or at positions 1 to 10 and 251 to 260 by substitution and at positions 448 to 468 by deletion.

These promoter modifications lead to improved product expression properties, in particular it leads to an increased expression rate. In particular the multiple modifications according to the present invention are particularly advantageous. If these multiple modifications are carried out at the defined positions, the properties of the promoter can be further improved.

According to a particularly preferred embodiment of the present invention the promoter comprising or consisting of a nucleic acid sequence having at least 80% identity to SEQ ID No. 4 is modified within at least one region selected from the group consisting of position 1 to 20, position 151 to 170 and position 171 to 190, preferably at position 1 to 20, position 161 to 170 and position 171 to 180. In doing so, it is even more preferred that the promoter comprising or consisting of a nucleic acid sequence having at least 80% identity to SEQ ID No. 4 is simultaneously modified at positions 161 to 170 and 171 to 180; or 11 to 20, 161 to 170 and 171 to 180.

It turned out that modifications of the aforementioned regions of the PDH promoter as defined result in a modified promoter showing an increased transcription rate of a nucleic acid sequence operably linked thereto compared to the wild-type promoter.

In order to increase the transcription rate further, the promoter comprising or consisting of a nucleic acid sequence having at least 80% identity to SEQ ID No. 4 is modified in a specific region with the nucleic acid sequence AAA. The properties of the promoter thus can be further improved by performing multiple modifications at specific positions with nucleic sequence AAA.

According to an especially preferred embodiment of the present invention the promoter comprising or consisting of a nucleic acid sequence having at least 80% identity to SEQ ID No. 4 is modified with nucleic acid AAA within at least one promoter region selected from the group consisting of position 235 to 237 and position 349 to 351.

According to a particularly preferred embodiment of the present invention the promoter comprising or consisting of a nucleic acid sequence having at least 80% identity to SEQ ID No. 4 is modified, preferably substituted, with nucleic acid AAA within at least one promoter region selected from the group consisting of position 37 to 39, position 103 to 105, position 109 to 111, position 178 to 180, position 235 to 237 and position 349 to 351.

According to an especially preferred embodiment of the present invention the promoter comprising or consisting of a nucleic acid sequence having at least 80% identity to SEQ ID No. 4 is modified within at least one promoter region selected from the group consisting of position 161 to 170, position 171 to 180, position 37 to 39, position 103 to 105, position 109 to 111, position 121 to 130, position 178 to 180, position 235 to 237 and position 349 to 351, whereby position 121 to 130, 161 to 170 and position 171 to 180 is preferably modified with nucleic acid GATAX₁X₂X₃X₄X₅X₆(X₇)ₘ and position 37 to 39, position 103 to 105, position 109 to 111, position 178 to 180, position 235 to 237 and position 349 to 351 are preferably modified with nucleic acid AAA.

The present invention also relates to a promoter variant obtainable by a method described in the invention. The promoter thus produced has improved properties compared to the original promoter from which it has been derived from. The promoter variant according to the invention preferably shows a higher transcription rate of a nucleic acid sequence operably linked thereto compared to the original promoter. The promoter variant according to the invention can be used in all areas in which a high level of protein production is desired.

Promotors produced by and/or being identified using the process of the present invention have improved properties compared to their respective original promotors. Preferably, these promoters have an increased transcription rate, in particular they show a higher transcription rate under methanol-inducing conditions and/or under de-repressed conditions.

According to a particularly preferred embodiment of the present invention these promoter variant comprises or consists of a nucleic acid sequence selected from the group of SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23 and SEQ ID No. 24.
SEQ ID No. 5: 
ATCCTTTTAGCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGGTCGGCATTACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTCTCGCACGAGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGTT
TCATTATCCCAGCGGGGTGATAGCCTCTGTTGCTCATCAGGCCAAAATCATATATAAGCTGT
AGACCCAGCACTTCAATTACTTGAAATTCACCATAACACTTGCTCTAGTCAAGACTTACAAT
TAAA
SEQ ID No. 6: 
TAATCGAACTCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGATAACCGTGACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTCTCGCACGAGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGTT
TCATTATCCCAGCGGGGTGATAGCCTCTGTTGCTCATCAGGCCAAAATCATATATAAGCTGT
AGACCCAGCACTTCAATTACTTGAAATTCACCATAACACTTGCTCTAGTCAAGACTTACAAT
TAAA
SEQ ID No. 7: 
TAATCGAACTCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGGTCGGCATTACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTGATAACCGTGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGTT
TCATTATCCCAGCGGGGTGATAGCCTCTGTTGCTCATCAGGCCAAAATCATATATAAGCTGT
AGACCCAGCACTTCAATTACTTGAAATTCACCATAACACTTGCTCTAGTCAAGACTTACAAT
TAAA
SEQ ID No. 8: 
TAATCGAACTCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGGTCGGCATTACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTCTCGCACGAGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGGA
TAACCGTGCCAGCGGGGTGATAGCCTCTGTTGCTCATCAGGCCAAAATCATATATAAGCTGT
AGACCCAGCACTTCAATTACTTGAAATTCACCATAACACTTGCTCTAGTCAAGACTTACAAT
TAAA
SEQ ID No. 9: 
TAATCGAACTCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGGTCGGCATTACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTCTCGCACGAGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGTT
TCATTATCCCAGCGGGGTGATAGCCTCTGTTGCTCATCAGGCCAAAATCATATATAAGCTGT
AGACCCAGCACTTCAATTACTTGAAATTCACCATAAGATAACCGTGAGTCAAGACTTACAAT
TAAA
SEQ ID No. 10: 
TAATCGAACTCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGATAACCGTGACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTGATAACCGTGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGTT
TCATTATCCCAGCGGGGTGATAGCCTCTGATAACCGTGAGGCCAAAATCATATATAAGCTGT
AGACCCAGCACTTCAATTACTTGAAATTCACCATAACACTTGCTCTAGTCAAGACTTACAAT
TAAA
SEQ ID No. 11: 
TAATCGAACTCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGATAACCGTGACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTGATAACCGTGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGTT
TCATTATCCCAGCGGGGTGATAGCCTCTGTTGCTCATCAGGCCAAAATCATATATAAGCTGT
AGACCCAGCACTTCAATTACTTGAAATTCACCATAAGATAACCGTGAGTCAAGACTTACAAT
TAAA
SEQ ID No. 12: 
ATCCTTTTAGCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGATAACCGTGACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTGATAACCGTGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGTT
TCATTATCCCAGCGGGGTGATAGCCTCTGTTGCTCATCAGGCCAAAATCATATATAAGCTGT
AGACCCAGCACTTCAATTACTTGAAATTCACCATAACACTTGCTCTAGTCAAGACTTACAAT
TAAA
SEQ ID No. 13: 
ATCCTTTTAGCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
 
TGTTCCGATAACCGTGACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTGATAACCGTGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGGA
TAACCGTGCCAGCGGGGTGATAGCCTCTGTTGCTCATCAGGCCAAAATCATATATAAGCTGT
AGACCCAGCACTTCAATTACTTGAAATTCACCATAACACTTGCTCTAGTCAAGACTTACAAT
TAAA
SEQ ID No. 14: 
ATCCTTTTAGCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGATAACCGTGACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTGATAACCGTGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGTT
TCATTATCCCAGCGGGGTGATAGCCTCTGTTGCTCATCAGGCCAAAATCATATATAAGCTGT
AGACCCAGCACTTCAATTACTTGAAATTCACCATAAGATAACCGTGAGTCAAGACTTACAAT
TAAA
SEQ ID No. 15: 
ATCCTTTTAGCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGATAACCGTGACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTGATAACCGTGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGGA
TAACCGTGCCAGCGGGGTGATAGCCTCTGATAACCGTGAGGCCAAAATCATATATAAGCTGT
AGACCCAGCACTTCAATTACTTGAAATTCACCATAAGATAACCGTGAGTCAAGACTTACAAT
TAAA
SEQ ID No. 16: 
CTTCAGTAAGGATAACCGTGTTGGTGACGATCACAGCTATGATGTAATAGAAATTGCTAAGC
AATTGTGAGGTGTGATGTATTTTGCAGAGCAATTGTGCGGTACAACGGGGTGTTATTGTCTT
CACAAGGCATTTATTGCGAATTTCGTAGTTGAAAGAATATTTTAGCACAGGGTGCTTGACCC
CTATTGTTGCTCGCTAAACCATGATTGCTAAATGATGACATAGCAATCACTTTACTAAGATT
 
GCTATAAGGACACCTTTCTTAGTATAAATGGACACTCTTTTCCCCTGCTAAACTTCTTTTAT
TTTTCACACTTAAACAGTTACAAAACACAAACACAACTAGAA
SEQ ID No. 17: 
CTTCAGTAAGAGAAGGAAGCTTGGTGACGATCACAGCTATGATGTAATAGAAATTGCTAAGC
AATTGTGAGGTGTGATGTATTTTGCAGAGCAATTGTGCGGTACAACGGGGTGTTATTGGATA
ACCGTGGCATTTATTGCGAATTTCGTAGTTGAAAGAATATTTTAGCACAGGGTGCTTGACCC
CTATTGTTGCTCGCTAAACCATGATTGCTAAATGATGACATAGCAATCACTTTACTAAGATT
GCTATAAGGACACCTTTCTTAGTATAAATGGACACTCTTTTCCCCTGCTAAACTTCTTTTAT
TTTTCACACTTAAACAGTTACAAAACACAAACACAACTAGAA
SEQ ID No. 18: 
CTTCAGTAAGAGAAGGAAGCTTGGTGACGATCACAGCTATGATGTAATAGAAATTGCTAAGC
AATTGTGAGGTGTGATGTATTTTGCAGAGCAATTGTGCGGTACAACGGGGTGTTATTGTCTT
CACAAGGCATTTATTGCGAATTTCGTAGTTGAAAGAGATAACCGTGACAGGGTGCTTGACCC
CTATTGTTGCTCGCTAAACCATGATTGCTAAATGATGACATAGCAATCACTTTACTAAGATT
GCTATAAGGACACCTTTCTTAGTATAAATGGACACTCTTTTCCCCTGCTAAACTTCTTTTAT
TTTTCACACTTAAACAGTTACAAAACACAAACACAACTAGAA
SEQ ID No. 19: 
CTTCAGTAAGAGAAGGAAGCTTGGTGACGATCACAGCTATGATGTAATAGAAATTGCTAAGC
AATTGTGAGGTGTGATGTATTTTGCAGAGCAATTGTGCGGTACAACGGGGTGTTATTGTCTT
CACAAGGCATTTATTGCGAATTTCGTAGTTGAAAGAATATTTTAGCGATAACCGTGTGACCC
CTATTGTTGCTCGCTAAACCATGATTGCTAAATGATGACATAGCAATCACTTTACTAAGATT
GCTATAAGGACACCTTTCTTAGTATAAATGGACACTCTTTTCCCCTGCTAAACTTCTTTTAT
TTTTCACACTTAAACAGTTACAAAACACAAACACAACTAGAA
SEQ ID No. 20: 
CTTCAGTAAGAGAAGGAAGCTTGGTGACGATCACAGCTATGATGTAATAGAAATTGCTAAGC
AATTGTGAGGTGTGATGTATTTTGCAGAGCAATTGTGCGGTACAACGGGGTGTTATTGTCTT
CACAAGGCATTTATTGCGAATTTCGTAGTTGAAAGAGATAACCGTGGATAACCGTGTGACCC
CTATTGTTGCTCGCTAAACCATGATTGCTAAATGATGACATAGCAATCACTTTACTAAGATT
GCTATAAGGACACCTTTCTTAGTATAAATGGACACTCTTTTCCCCTGCTAAACTTCTTTTAT
TTTTCACACTTAAACAGTTACAAAACACAAACACAACTAGAA
SEQ ID No. 21: 
CTTCAGTAAGGATAACCGTGTTGGTGACGATCACAGCTATGATGTAATAGAAATTGCTAAGC
AATTGTGAGGTGTGATGTATTTTGCAGAGCAATTGTGCGGTACAACGGGGTGTTATTGTCTT
CACAAGGCATTTATTGCGAATTTCGTAGTTGAAAGAGATAACCGTGGATAACCGTGTGACCC
CTATTGTTGCTCGCTAAACCATGATTGCTAAATGATGACATAGCAATCACTTTACTAAGATT
GCTATAAGGACACCTTTCTTAGTATAAATGGACACTCTTTTCCCCTGCTAAACTTCTTTTAT
TTTTCACACTTAAACAGTTACAAAACACAAACACAACTAGAA
SEQ ID No. 22: 
CTTCAGTAAGAGAAGGAAGCTTGGTGACGATCACAGCTATGATGTAATAGAAATTGCTAAGC
AATTGTGAGGTGTGATGTATTTTGCAGAGCAATTGTGCGGTACAACGGGGTGTTATTGTCTT
CACAAGGCATTTATTGCGAATTTCGTAGTTGAAAGAATATTTTAGCACAGGGTAAATGACCC
CTATTGTTGCTCGCTAAACCATGATTGCTAAATGATGACATAGCAATCAAATTACTAAGATT
GCTATAAGGACACCTTTCTTAGTATAAATGGACACTCTTTTCCCCTGCTAAACTTCTTTTAT
TTTTCACACTTAAACAGTTACAAAACACAAACACAACTAAAA
SEQ ID No. 23: 
CTTCAGTAAGAGAAGGAAGCTTGGTGACGATCACAGAAATGATGTAATAGAAATTGCTAAGC
AATTGTGAGGTGTGATGTATTTTGCAGAGCAATTGTGCGGAAAAACAAAGTGTTATTGTCTT
CACAAGGCATTTATTGCGAATTTCGTAGTTGAAAGAATATTTTAGCACAGGGTAAATGACCC
CTATTGTTGCTCGCTAAACCATGATTGCTAAATGATGACATAGCAATCAAATTACTAAGATT
GCTATAAGGACACCTTTCTTAGTATAAATGGACACTCTTTTCCCCTGCTAAACTTCTTTTAT
TTTTCACACTTAAACAGTTACAAAACACAAACACAACTAAAA
SEQ ID No. 24:

CTTCAGTAAGAGAAGGAAGCTTGGTGACGATCACAGCTATGATGTAATAGAAATTGCTAAGC
AATTGTGAGGTGTGATGTATTTTGCAGAGCAATTGTGCGGTACAACGGGGTGTTATTGTCTT
CACAAGGCATTTATTGCGAATTTCGTAGTTGAAAGAGATAACCGTGGATAACCGTGTGACCC
CTATTGTTGCTCGCTAAACCATGATTGCTAAATGATGACATAGCAATCAAATTACTAAGATT
GCTATAAGGACACCTTTCTTAGTATAAATGGACACTCTTTTCCCCTGCTAAACTTCTTTTAT
TTTTCACACTTAAACAGTTACAAAACACAAACACAACTAAAA

The present invention further relates to a nucleic acid molecule or vector comprising a promoter variant according to the invention.

The nucleic acid molecule of the present invention may be operably linked to a nucleic acid sequence encoding for a polypeptide or protein. These nucleic acid molecules or the promoter variants of the present invention may be part of a vector, including plasmids, cosmids or Yeast Artificial Chromosomes (YACs) and modified viruses or virus particles.

These nucleic acid molecules and in particular these vectors may comprise further elements required for protein expression or replication within a host cell. Such elements are well known to a person skilled in the art.

According to an especially preferred embodiment of the present invention the promoter variant of the present invention is operably linked to a nucleic acid sequence encoding for a polypeptide or protein.

The present invention further relates to a host cell comprising a nucleic acid molecule or promoter variant according to the invention.

The nucleic acid molecule and/or the promoter of the present invention may be comprised within a host cell. The host cell can be used as a carrier for said nucleic acid and/or the promoter or can be functionally active therein. If the nucleic acid molecule and/or the promoter of the present invention is functionally active, the host cell can be used to express a protein or polypeptide of interest under the control of the promoter of the present invention.

According to a particularly preferred embodiment of the present invention the host cell is a fungal cell, preferably from the phylum Ascomycota, more preferably from the genus Ko*magataella,* more preferably a *Komagataella phaffii* cell.

The present invention further relates to a method for producing a polypeptide or protein of interest comprising the step of cultivating a host cell according to the invention. Methods for cultivating cells and for activating the promoter variants of the present invention to overexpress proteins and polypeptides are well known to a person skilled in the art and depend on the modified promoter.

The present invention is further illustrated by the following examples without being restricted thereto.

### EXAMPLES

In the present examples, two promoters, namely catalase promoter PCTA1 (SEQ ID No. 3) of *K. phaffii* and heat shock protein 12 promoter PDH (SEQ ID No. 4), have been modified using the method of the present invention to identify promoter variants showing a higher expression rate than the wild-type promoters ("original promoters").

The promoter sequences have been modified by means of insertion, substitution, or deletion. The respective type and position of modification is indicated.

### Example 1: Influence of a single mutation on expression under de-repressed conditions

### Promoter PCTA1

The whole promoter sequence was systematically scanned using two different 10 bp long "neutral" sequences termed A (5'-ATCCTTTTAG-3'; SEQ ID No. 1) and B (5'-GATAACCGTG-3'; SEQ ID No. 2). By systematically exchanging every ten base pairs against these two A and B sequences overall 100 so called "scanning variants", named 1A/B to 50A/B with the numbering starting at the 5'end of the promoter, were generated.

In order to evaluate the promoter strength of the scanning variants the promoter and promoter variants have been operably linked to a nucleic acid sequence encoding for the enhanced green fluorescent protein (eGFP) (GenBank Acc. No. ADL66923) (Vogl et al. ACS Synth. Biol. 5(2016):172-186). The promoter activity was determined as relative fluorescence unit (RFU) normalized to cell density (OD₆₀₀) at different timepoints in the cultivation either upon carbon source depletion (indicated as de-repressed) or after methanol induction (indicated as induced). The RFU was then set in relation to the respective value of the original (i.e. unmodified) promoter sequence. Cultivations were done in microscale using 96-well cultivation plates. For transformations 1 µg Smal linearized DNA was used for transformation of electrocompetent CBS7435 Δku70 cells and following initial selection on YPD_{Zeo}, single colonies were used for inoculation of microscale cultures.

Following Weis et al. (FEMS Yeast Res 5(2004):179-89) cultivations with yeast cells comprising promoter PCTA1 and (scanning) variants thereof were done in buffered minimal media using 1% glucose as carbon source at 28°C, 320 rpm for 60h, followed by methanol induction to 0.5% MeOH for 38h.

Cultivations with yeast cells comprising promoter PDH and (scanning) variants thereof were done in buffered minimal media using 1% glycerol as carbon source at 28°C, 320 rpm for 48h, followed by glycerol pulsing to 0.25% for 96h.

In the following tables the de-repressed and induced activities of the variants given by the RFU normalized to OD₆₀₀ in relation to RFU obtained by using the original promoter sequences are listed.

**Table 1: Original promoter sequence was SEQ ID No. 3 [PCTA1]**

| **SEQ ID No.** | **Position substitution** | **Sequence inserted by substitution** | **NA derepressed 48h** | **NA derepressed 60h** | **NA induced 98h** |
|---|---|---|---|---|---|
| 5 [1A] | 1-10 | ATCCTTTTAG | 1.48 | 1.48 | 1.15 |
| 6 [14B] | 131-140 | GATAACCGTG | 1.65 | 1.69 | 1.28 |
| 7 [26B] | 251-260 | GATAACCGTG | 1.63 | 1.8 | 1.24 |
| 8 [38B] | 371-380 | GATAACCGTG | | 1.08 | 1.42 |
| 25 [41B] | 401-410 | GATAACCGTG | | 0.85 | 1.42 |
| 9 [48B] | 471-480 | GATAACCGTG | | 1.06 | 1.31 |

SEQ ID No. 25:

TAATCGAACTCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGGTCGGCATTACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTCTCGCACGAGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGTT

TCATTATCCCAGCGGGGTGATAGCCTCTGATAACCGTGAGGCCAAAATCATATATAAGCTGT
AGACCCAGCACTTCAATTACTTGAAATTCACCATAACACTTGCTCTAGTCAAGACTTACAAT
TAAA

Table 1 clearly shows that substitution variants according to the invention have a higher promoter activity than the original *Komagataella phaffii* PCTA1 promoter. From the measurements can also be recognised that the modifications within the preferred regions according to the invention lead to an even higher promoter activity than those outside these regions. Changes under derepressed conditions are higher than under induced conditions. In addition to these substitution variants the PCTA1 promoter has also been modified by deleting putative TFBS. For these variants changes in activity were not as pronounced as for the "scanning variants", as most of them showed fluorescence levels within 20% of the parental promoter. Only three variants reached higher values of around 140%-180% compared to the parental PCTA1 under derepressed and induced conditions, respectively. Following variants could be used for a combinatory approach together with "scanning variants": #1, #4 and #53* (see Table 2).

**Table 2: Deletion variants of PCTA1 (SEQ ID No. 3)**

| **SEQ ID No.** | **Start-End deletion** | **De-repressed Activity/PCTAl** | **Induced Activity/PCTAl** |
|---|---|---|---|
| 26 [#1] | 2-16 | 1.38 | 1.36 |
| 27 [#4] | 28-40 | 0.99 | 1.05 |
| 28 [#53*] | 448-468 | 1.57 | 1.12 |
| 29 [MM13b] | 89-101, 137-138, 162, 176 | 1.08 | 1.20 |

| | | | |
|---|---|---|---|
| 53* contains an additional randomly introduced A to T exchange at position 408. | | | |

SEQ ID No. 26: 
TGCGGTTCTCCTGTAACCTTAATTGTAGCATAGATCACTTAAATAAACTCATGGCCTGACAT
CTGTACACGTTCTTATTGGTCTTTTAGCAATCTTGAAGTCTTTCTATTGTTCCGGTCGGCAT
 
TACCTAATAAATTCGAATCGAGATTGCTAGTACCTGATATCATATGAAGTAATCATCACATG
CAAGTTCCATGATACCCTCTACTAATGGAATTGAACAAAGTTTAAGCTTCTCGCACGAGACC
GAATCCATACTATGCACCCCTCAAAGTTGGGATTAGTCAGGAAAGCTGAGCAATTAACTTCC
CTCGATTGGCCTGGACTTTTCGCTTAGCCTGCCGCAATCGGTAAGTTTCATTATCCCAGCGG
GGTGATAGCCTCTGTTGCTCATCAGGCCAAAATCATATATAAGCTGTAGACCCAGCACTTCA
ATTACTTGAAATTCACCATAACACTTGCTCTAGTCAAGACTTACAATTAAA
SEQ ID No. 27: 
TAATCGAACTCCGAATGCGGTTCTCCTTAGCATAGATCACTTAAATAAACTCATGGCCTGAC
ATCTGTACACGTTCTTATTGGTCTTTTAGCAATCTTGAAGTCTTTCTATTGTTCCGGTCGGC
ATTACCTAATAAATTCGAATCGAGATTGCTAGTACCTGATATCATATGAAGTAATCATCACA
TGCAAGTTCCATGATACCCTCTACTAATGGAATTGAACAAAGTTTAAGCTTCTCGCACGAGA
CCGAATCCATACTATGCACCCCTCAAAGTTGGGATTAGTCAGGAAAGCTGAGCAATTAACTT
CCCTCGATTGGCCTGGACTTTTCGCTTAGCCTGCCGCAATCGGTAAGTTTCATTATCCCAGC
GGGGTGATAGCCTCTGTTGCTCATCAGGCCAAAATCATATATAAGCTGTAGACCCAGCACTT
CAATTACTTGAAATTCACCATAACACTTGCTCTAGTCAAGACTTACAATTAAA
SEQ ID No. 28: 
TAATCGAACTCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGGTCGGCATTACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTCTCGCACGAGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGTT
TCATTATCCCAGCGGGGTGATAGCCTCTGTTGCTCTTCAGGCCAAAATCATATATAAGCTGT
AGACCCAGCACTTAACACTTGCTCTAGTCAAGACTTACAATTAAA
SEQ ID No. 29:

TAATCGAACTCCGAATGCGGTTCTCCTGTAACCTTAATTGTAGCATAGATCACTTAAATAAA
CTCATGGCCTGACATCTGTACACGTTTAGCAATCTTGAAGTCTTTCTATTGTTCCGGTCGGT
TACCTAATAAATTCGAATCGAGTTGCTAGTACCTGTATCATATGAAGTAATCATCACATGCA
AGTTCCATGATACCCTCTACTAATGGAATTGAACAAAGTTTAAGCTTCTCGCACGAGACCGA
ATCCATACTATGCACCCCTCAAAGTTGGGATTAGTCAGGAAAGCTGAGCAATTAACTTCCCT
CGATTGGCCTGGACTTTTCGCTTAGCCTGCCGCAATCGGTAAGTTTCATTATCCCAGCGGGG

TGATAGCCTCTGTTGCTCATCAGGCCAAAATCATATATAAGCTGTAGACCCAGCACTTCAAT
TACTTGAAATTCACCATAACACTTGCTCTAGTCAAGACTTACAATTAAA

The results obtained by screening substitution variants using HSP12 promoter PDH are shown in Table 3.

**Table 3: Original promoter sequence is SEQ ID No. 4 [PDH]**

| **SEQ ID No.** | **Start-End substitution** | **Sequence inserted by substitution** | **NA derepressed 48h** | **NA derepressed 72h** | **NA derepressed 96h** |
|---|---|---|---|---|---|
| 18 [V17] | 161-170 | GATAACCGTG | 1.5 | 1.87 | 1.4 |
| 19 [V18] | 171-180 | GATAACCGTG | 1.4 | 1.48 | 1.43 |
| 30 [V5] | 41-50 | GATAACCGTG | 1.01 | 1.02 | 1.03 |
| 31 [A60] | 178-180 | AAA | 1.4 | 1.1 | 1.1 |

SEQ ID No. 30: 
CTTCAGTAAGAGAAGGAAGCTTGGTGACGATCACAGCTATGATAACCGTGAAATTGCTAAGC
AATTGTGAGGTGTGATGTATTTTGCAGAGCAATTGTGCGGTACAACGGGGTGTTATTGTCTT
CACAAGGCATTTATTGCGAATTTCGTAGTTGAAAGAATATTTTAGCACAGGGTGCTTGACCC
CTATTGTTGCTCGCTAAACCATGATTGCTAAATGATGACATAGCAATCACTTTACTAAGATT
GCTATAAGGACACCTTTCTTAGTATAAATGGACACTCTTTTCCCCTGCTAAACTTCTTTTAT
TTTTCACACTTAAACAGTTACAAAACACAAACACAACTAGAA
SEQ ID No. 31:

CTTCAGTAAGAGAAGGAAGCTTGGTGACGATCACAGCTATGATGTAATAGAAATTGCTAAGC
AATTGTGAGGTGTGATGTATTTTGCAGAGCAATTGTGCGGTACAACGGGGTGTTATTGTCTT
CACAAGGCATTTATTGCGAATTTCGTAGTTGAAAGAATATTTTAGCACAGGGTAAATGACCC
CTATTGTTGCTCGCTAAACCATGATTGCTAAATGATGACATAGCAATCACTTTACTAAGATT
GCTATAAGGACACCTTTCTTAGTATAAATGGACACTCTTTTCCCCTGCTAAACTTCTTTTAT
TTTTCACACTTAAACAGTTACAAAACACAAACACAACTAGAA

From Table 3 it can be seen that also the single mutation variants according to the invention using the SED ID No. 4 [PDH] lead to a higher promoter activity than the original promoter sequence. Again, a higher activity can be achieved when the mutation is carried out within the preferred region.

### Example 2: Influence of multiple mutations on expression under de-repressed conditions

Multiple mutation variants were produced as described above. The cultivations using the catalase promoter SEQ ID No. 3 [PCTA1] were done using 1% glucose as carbon source at 28°C, 320 rpm for 60 hours for the double and triple mutation variants and using 0.5% glucose as carbon source at 28°C, 320 rpm for 60 hours, for the quadruple and sextuple mutation variants.

The cultivations using the heat shock protein promoter SEQ ID No. 4 [PDH] were done using 1% glycerol as carbon source at 28°C, 320 rpm for 48 hours, followed by glycerol pulsing to 0.25% for 94h.

The activities of the variants given by the RFU normalized to OD₆₀₀ in relation to the original promoter sequence are given in the following tables.

**Table 4: Original promoter sequence is SEQ ID No. 3 [PCTA1]**

| **SEQ ID No.** | **Start-End substitution** | **Sequence inserted by substitution** | **NA derepressed 48h** | **NA derepressed 60h** | **NA induced 98h** |
|---|---|---|---|---|---|
| 10 [14B,26B, 41B] | 131-140 | GATAACCGTG | 3.05 | 3.10 | 1.49 |
| | 251-260 | GATAACCGTG | | | |
| | 401-410 | GATAACCGTG | | | |
| 14 [1A, 14B, 2 6B, 48B] | 1-10 | ATCCTTTTAG | 3.01 | 3.95 | 1.85 |
| | 131-140 | GATAACCGTG | | | |
| | 251-260 | GATAACCGTG | | | |
| | 471-480 | GATAACCGTG | | | |
| 15 | 1-10 | ATCCTTTTAG | 3.35 | 3.5 | 2.5 |
| | 131-140 | GATAACCGTG | | | |
| | 251-260 | GATAACCGTG | | | |
| [1A,14B,2 6B,38B,41 B, 48B] | 371-380 | GATAACCGTG | | | |
| | 401-410 | GATAACCGTG | | | |
| | 471-480 | GATAACCGTG | | | |
| 11 [14B, 26B, 48B] | 131-140 | GATAACCGTG | 2.5 | 2.6 | 1.7 |
| | 251-260 | GATAACCGTG | | | |
| | 471-480 | GATAACCGTG | | | |
| 12 [1A,14B,2 6B] | 1-10 | ATCCTTTTAG | 2.9 | 2.7 | 1.6 |
| | 131-140 | GATAACCGTG | | | |
| | 251-260 | GATAACCGTG | | | |
| 13 [1A,14B,2 6B, 38B] | 1-10 | ATCCTTTTAG | 3.0 | 3.01 | 2.00 |
| | 131-140 | GATAACCGTG | | | |
| | 251-260 | GATAACCGTG | | | |
| | 371-380 | GATAACCGTG | | | |
| 32 [38B,41B, 48B] | 371-380 | GATAACCGTG | 1.05 | 1.01 | 1.9 |
| | 401-410 | GATAACCGTG | | | |
| | 471-480 | GATAACCGTG | | | |

SEQ ID No. 32:

TAATCGAACTCCGAATGCGGTTCTCCTGTAACCTTAATTGTAGCATAGATCACTTAAATAAA
CTCATGGCCTGACATCTGTACACGTTCTTATTGGTCTTTTAGCAATCTTGAAGTCTTTCTAT
TGTTCCGGTCGGCATTACCTAATAAATTCGAATCGAGATTGCTAGTACCTGATATCATATGA
AGTAATCATCACATGCAAGTTCCATGATACCCTCTACTAATGGAATTGAACAAAGTTTAAGC
TTCTCGCACGAGACCGAATCCATACTATGCACCCCTCAAAGTTGGGATTAGTCAGGAAAGCT
GAGCAATTAACTTCCCTCGATTGGCCTGGACTTTTCGCTTAGCCTGCCGCAATCGGTAAGGA
TAACCGTGCCAGCGGGGTGATAGCCTCTGATAACCGTGAGGCCAAAATCATATATAAGCTGT
AGACCCAGCACTTCAATTACTTGAAATTCACCATAAGATAACCGTGAGTCAAGACTTACAAT
TAAA

Table 4 shows that multiple mutation variants have a higher promoter activity than the original promoter. Generally, multiple mutation lead to a higher expression than single mutation. As for single mutation variants, the changes in eGFP expression are greater under derepressed conditions.

**Table 5: Original promoter sequence is SEQ ID No. 4 [PDH]**

| **SEQ ID No.** | **Start-End substitution** | **Sequence inserted by substitution** | **NA derepressed 46h** | **NA derepressed 70h** | **NA derepressed 94h** |
|---|---|---|---|---|---|
| 20 [V17,V18] | 161-170 | GATAACCGTG | 1.3 | 1.35 | 1.12 |
| | 171-180 | GATAACCGTG | | | |
| 21 [V2,V17,V 18] | 11-20 | GATAACCGTG | 1.51 | 1.6 | 1.4 |
| | 161-170 | GATAACCGTG | | | |
| | 171-180 | GATAACCGTG | | | |
| 24 [A79,A117 ,V17,V18] | 235-237 | AAA | 2.1 | 2.3 | 1.98 |
| | 349-351 | AAA | | | |
| | 161-170 | GATAACCGTG | | | |
| | 171-180 | GATAACCGTG | | | |

From Table 5 it can be deduced that multiple mutations according to the invention lead to an even higher expression/promoter activity than the original promoter. Multiple mutation increases the activity further than single mutation. Especially under derepressed conditions a change in expression activity can be observed. When modifying the promoter sequence multiple times at the particularly preferred positions even higher expression activities can be achieved.

Further experiments were carried out to analyse the expression activity, using the lipase of Candida antarctica (CalB), the human growth hormone (HGH) or peroxygenase as secreted model proteins. The results are consistent with those of the experiments carried out with eGFP, so that for those proteins similar expression activities were found.

### Example 3: Scaleup of promoter variants with secreted model protein CalB

The performance of two promoter variants from example 2 was evaluated in medium scale cultivations using shake flasks. The tested promoter variants had SEQ ID No. 12 [1A,14B,26B] and SEQ ID No. 11 [14B,26B,48B]. In the following SEQ ID No. 12 [1A,14B,26B] is named #19 and SEQ ID No. 11 [14B,26B,48B] #26. As controls the parental *Komagataella phaffii* catalase promoter and a negative control were included. Representative for each promoter one average performing strain has been selected.

The cultivations were conducted in two manners, one time with BMD 0.5% and methanol induction and one time with BMG 0.5% and glycerol feeding discs to establish a derepressed state. Cultivations were done in biological duplicates and samples are taken after 4, 17, 24, 42 and 65 hours. The process was monitored online using the SFR vario system (PreSens Precision Sensing GmbH, Regensburg, Germany) so that methanol induction and the addition of feed discs can be done upon depletion of initial carbon source after 20 hours. For each timepoint enzyme activity was set in relation to the respective value of the parental promoter.

Only supernatants of sampling points 24 hours and onward showed lipase activity, before no lipase activity could be determined.

In the following table the activity measurements are listed. Volumetric units are normalized to the wildtype and plotted against the variants.

| **Time [h]** | **NA derepressed #19** | **NA derepressed #26** | **NA induced #19** | **NA induced #26** |
|---|---|---|---|---|
| **24** | 1.40 | 1.05 | 4.45 | 3.00 |
| **42** | 2.10 | 1.45 | 2.50 | 1.60 |
| **65** | 2.90 | 1.70 | 2.30 | 1.50 |

As can be seen from the above table both variants show even better performance in medium scale cultivation than in microscale cultivation under both cultivation conditions.

### Example 4: Determination of expression level of a multiple mutation variant using qPCR

The mutation variant #19 from example 3 was further characterized for its expression level using qPCR (Abad S et al. Biotechnol J 5(2010):413-20). To do so, cell material was harvested at different timepoints of a shake flask cultivation (16, 20, 22, 44, 46 and 68 hours), lipase activity is determined and RNA is isolated and used for qPCR. By using the comparative ΔΔCT method the relative changes in gene expression were calculated for the analysed *P. pastoris* reporter strains. The outcome is presented as the fold change in gene expression normalised to an internal reference gene (housekeeping gene) and relative to an untreated control (calibrator). ACT1 was used as house keeping gene to normalize target RNA input amounts and as calibrator variant #19 after 16 hours was used.

Fig. 1 shows lipase activity (A) and fold changes in the target gene (CalB) (B) for variant #19, the original promoter and reference promoter PDF (Vogl et al. MB Expr 10(2020):38)(HpFMD promoter fragment) with two different cultivation strategies, namely MeOH induction and derepression by glycerol feed beads.

Lipase activity measurements (11A) verify the previously observed performance of the variant #19. Under methanol induced conditions variant #19 showed higher activity than the original and the PDF promoter. For prolonged de-repression using glycerol feed beads the variant #19 and the PDF performed equally well.

Under derepressed conditions with glycerol variant #19 shows slightly higher amounts of RNA compared to PDF. The overall highest fold change can be seen for variant #19 after 68h, implying that potentially even higher CalB activities than determined after 68h could be reached in further experiments.

## Claims

1. A method for modifying a promoter to obtain a promoter variant comprising the steps of:
a. providing a promoter,
b. modifying the promoter
- by inserting at least one nucleic acid sequence selected from the group consisting of GATAX₁X₂X₃X₄X₅X₆ (X₇) ₘ, ATCCTTTTAG (SEQ ID No. 1) and AAA into the promoter and/or
- by substituting nucleotides of the promoter sequence to introduce at least one nucleic acid sequence selected from the group consisting of GATAX₁X₂X₃X₄X₅X₆(X₇)m, ATCCTTTTAG (SEQ ID No. 1) and AAA into the promoter,
wherein X₁, X₂, X₃, X₄, X₅, X₆, and X₇ are nucleotides independently selected from the group consisting of A, C, G and T, and wherein m is an integer between 0 and 10.

2. The method according to claim 1, wherein X₁ is A, X₂ is C, X₃ is C, X₄ is G, X₅ is T and X₆ is G.

3. The method according to claim 1 or 2, wherein the at least one nucleic acid sequence consists of GATAACCGTG (SEQ ID No. 2).

4. The method according to any of claims 1 to 3, wherein the promoter is a eukaryotic promoter, preferably a fungal promoter, more preferably a yeast promoter, more preferably a methylotrophic yeast promoter.

5. The method according to any of claims 1 to 4, wherein the promoter comprises or consists of a nucleic acid sequence having at least 80% identity, preferably at least 90% identity, more preferably at least 95% identity, to SEQ ID No. 3 or SEQ ID No. 4.

6. The method according to claim 5, wherein the promoter comprising or consisting of a nucleic acid sequence having at least 80% identity to SEQ ID No. 3 is modified within at least one promoter region selected from the group consisting of position 1 to 20, position 131 to 150, position 251 to 270, position 371 to 390, position 401 to 410 and position 461 to 480 of SEQ ID No. 3.

7. The method according to claim 5 or 6, wherein the promoter comprising or consisting of a nucleic acid sequence having at least 80% identity to SEQ ID No. 3 is modified within at least one promoter region selected from the group consisting of position 1 to 10, position 131 to 140, position 251 to 260, position 371 to 380, position 401 to 410 and position 471 to 480 of SEQ ID No. 3 wherein position 1 to 10 of SEQ ID No. 3 is preferably modified with ATCCTTTTAG (SEQ ID No. 1) and positions 131 to 140, 251 to 260, 371 to 380, 401 to 410 and 471 to 480 of SEQ ID No. 3 are preferably modified with GATAX₁X₂X₃X₄X₅X₆(X₇)ₘ.

8. The method according to any of claims 5 to 7, wherein the promoter comprising or consisting of a nucleic acid sequence having at least 80% identity to SEQ ID No. 3 is modified at positions 1 to 10, 131 to 140 and 251 to 260; or 131 to 140, 251 to 260 and 401 to 410; or 131 to 140, 251 to 260 and 471 to 480; or 1 to 10, 131 to 140, 251 to 260 and 371 to 380; or 1 to 10, 131 to 140, 251 to 260 and 471 to 480; or 1 to 10, 131 to 140, 251 to 260, 371 to 380, 401 to 410 and 471 to 480 of SEQ ID No. 3, preferably at positions 1 to 10, 131 to 140 and 251 to 260 or 1 to 10, 131 to 140, 251 to 260, 371 to 380, 401 to 410 and 471 to 480 of SEQ ID No. 3.

9. The method according to any of claims 5 to 8, wherein one or more, preferably all, nucleotides within positions 89 to 101, 137, 138, 162, 176 and/or 448 to 468 of the promoter comprising or consisting of a nucleic acid sequence having at least 80% identity to SEQ ID No. 3 are deleted.

10. The method according to claim 5, wherein the promoter comprising or consisting of a nucleic acid sequence having at least 80% identity to SEQ ID No. 4 is modified within at least one promoter region selected from the group consisting of position 1 to 20, position 151 to 170, preferably position 161 to 170, and position 171 to 190, preferably position 171 to 180.

11. The method according to claim 10 or 16, wherein the promoter comprising or consisting of a nucleic acid sequence having at least 80% identity to SEQ ID No. 4 is modified at positions 161 to 170 and 171 to 180; or 11 to 20, 161 to 170 and 171 to 180.

12. The method according to claim 5, 10 or 11, wherein the promoter comprising or consisting of a nucleic acid sequence having at least 80% identity to SEQ ID No. 4 is modified with nucleic acid AAA within at least one promoter region selected from the group consisting of position 37 to 39, position 103 to 105, position 109 to 111, position 178 to 180, position 235 to 237 and position 349 to 351 of SEQ ID No. 4.

13. A promoter variant obtainable by a method according to any one of claims 1 to 12.

14. The promoter variant according to claim 13, wherein the promoter variant comprises or consists of a nucleic acid sequence selected from the group of SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23 and SEQ ID No. 24.

15. A nucleic acid molecule or vector comprising a promoter variant according to claim 13 or 14.

16. The nucleic acid molecule or vector according to claim 15, wherein the promoter variant is operably linked to a nucleic acid sequence encoding for a polypeptide or protein.

17. A host cell comprising a nucleic acid molecule or promoter variant according to any one of claims 13 to 16.

18. The host cell according to claim 17, wherein the host cell is a fungal cell, preferably from the phylum Ascomycota, more preferably from the genus *Komagataella,* more preferably a *Komagataella phaffii* cell.

19. A method for producing a polypeptide or protein comprising the step of cultivating a host cell according to claim 17 or 18.
